**Europäisches Patentamt**

**European Patent Office**

**Office européen des brevets**

(19)

(11) Veröffentlichungsnummer: **0 271 885 B1**

# (12) EUROPÄISCHE PATENTSCHRIFT

(45) Veröffentlichungstag der Patentschrift: **06.05.92**

(51) Int. Cl.⁵: **A61K 35/50**, A61K 37/02

(21) Anmeldenummer: **87118598.9**

(22) Anmeldetag: **15.12.87**

Teilanmeldung 91112381.8 eingereicht am 15/12/87.

(54) **Arzneimittel enthaltend das Gewebeprotein PP4, Verfahren zur Herstellung von PP4 und zu seiner Pasteurisierung sowie die Verwendung von PP4.**

(30) Priorität: **18.12.86 DE 3643182**

(43) Veröffentlichungstag der Anmeldung:
**22.06.88 Patentblatt 88/25**

(45) Bekanntmachung des Hinweises auf die Patenterteilung:
**06.05.92 Patentblatt 92/19**

(84) Benannte Vertragsstaaten:
**AT BE CH DE ES FR GB GR IT LI LU NL SE**

(56) Entgegenhaltungen:
**EP-A- 0 123 307**
**EP-A- 0 217 341**

**EUROPEAN JOURNAL OF BIOCHEMISTRY, Band 151, 16. September 1985, Seiten 625-629, FEBS; C.P.M. REUTELINGSPERGER et al.: "Isolation and partial purification of a novel anticoagulant from arteries of human umbilical cord"**

(73) Patentinhaber: **BEHRINGWERKE Aktiengesellschaft**
**Postfach 1140**
**W-3550 Marburg 1(DE)**

(72) Erfinder: **Löbermann, Hartmut, Dr.**
**Giessener Strasse 50**
**W-3556 Weimar(DE)**
Erfinder: **Bornmann, Christiane**
**Steinweg 32**
**W-3573 Gemünden Wohra(DE)**

(74) Vertreter: **Klein, Otto, Dr. et al**
**Hoechst AG Zentrale Patentabteilung Postfach 80 03 20**
**W-6230 Frankfurt am Main 80(DE)**

**Beschreibung**

Die Erfindung betrifft ein Mittel enthaltend das Gewewbeprotein PP4 zur Hemmung der Blutgerinnung sowie ein Verfahren zu seiner Herstellung.

In DE 33 15 000 A 1 (USP 4,507,229) ist das Glycoprotein PP4 beschrieben, das beispielsweise in Placenta gefunden wird. PP4 besitzt ein Molekulargewicht von 35 000 ± 5 000 Dalton und einen isoelektrischen Bereich von pH 4.85 ± 0.15.

Blutgerinnungshemmende Proteine lassen sich aufgrund ihrer Wirkungsweise in mehrere Gruppen einteilen. Sowohl die Gruppe der Proteinase-Inhibitoren, wie beispielsweise Antithrombin III, als auch der Proteinasen, beispielsweise aktiviertes Protein C, bringen zum Teil nachteilige Eigenschaften für eine gerinnungshemmende Therapie mit sich, da sie entweder erst auf der Stufe der aktivierten Gerinnungsenzyme wirken oder aber durch eine Proteolyse von Gerinnungsfaktoren diese desaktivieren, sie "verbrauchen".

Aufgabe der Erfindung war daher ein die Blutgerinnung hemmendes Mittel zur Verfügung zu stellen, das die Blutgerinnung verhindern kann, ohne zu einem Verbrauch von Gerinnungsfaktoren zu führen.

In DE 35 33 516 A 1, Seiten 6 bis 8, sind blutgerinnungshemmende Proteine beschrieben, für die ein isoelektrischer Bereich von 4.4 - 4.6 angegeben ist. Diese Proteine werden aus der Intimaschicht größerer Gefäße, beispielsweise Aorta oder Nabelschnur, isoliert.

In Acta Obst. Gynaec. Jpn. 36, No. 12, 2583-2592 (1984) ist ebenfalls ein gerinnungshemmendes Protein aus Placenta beschrieben, das ein Molekulargewicht von 45 000 Dalton hat. In DE 33 15 000 A1 (USP 4,507,229) sind auch die folgenden Eigenschaften von PP4 beschrieben:
eine elektrophoretische Beweglichkeit im Bereich zwischen der von $alpha_1$ und $alpha_2$ Globulinen; ein Sedimentationskoeffizient

$$s^0_{20,w}$$

von 3,3 ± 0,2 S; ein Extinktionskoeffizient

$$E^{1\%}_{1cm}$$

(280 nm) von 5,9 ± 0,6; ein Kohlenhydratanteil von 2,4 ± 0,94% (g/100 g) (Mannose 0,3 ± 0,2 %, Galactose 0,4 ± 0,2 %, Xylose 0,1 ± 0,04 %, Glukose 0,2 ± 0,1 %, Glukosamin 1,0 + 2 %, Neuraminsäure 0,4 ± 0,2 %) und die folgenden Aminosäurenzusammensetzung:

| Aminosäure | Reste pro 100 Reste (mol %) | Variationskoeffizient |
|---|---|---|
| Lysin | 6,95 | 1,14 |
| Histidin | 0,97 | 17,4 |
| Arginin | 5,44 | 1,77 |
| Asparaginsäure | 11,41 | 1,68 |
| Threonin | 6,78 | 2,40 |
| Serin | 6,21 | 2,26 |
| Glutaminsäure | 12,25 | 0,43 |
| Prolin | 1,96 | 6,20 |
| Glycin | 6,68 | 3,83 |
| Alanin | 7,92 | 1,67 |
| Cystin 1/2 | 0,77 | 19,5 |
| Valin | 5,34 | 3,80 |
| Methionin | 1,98 | 6,00 |
| Isoleucin | 5,21 | 2,23 |
| Leucin | 11,50 | 0,45 |
| Tyrosin | 3,55 | 4,21 |
| Phenylalanin | 4,07 | 3,77 |
| Tryptophan | 0,93 | 23,9 |

Es wurde überraschenderweise gefunden, daß dieses Glycoprotein PP4 blutgerinnungshemmende Eigenschaften aufweist.

Gegenstand der Erfindung ist somit die Verwendung des Glykoproteins PP4 zur Hemmung der Blutgerinnung.

Gegenstand der Erfindung ist weiterhin ein Mittel zur Therapie oder Prophylaxe hämostatischer Störungen, das eine wirksame Menge PP4 und einen pharmazeutisch geeigneten Träger und/oder Stabilisator enthält.

Ein solches Mittel enthält in einer Lösung bevorzugt 0,001 - 100 mg PP4/ml Lösung. Es wird in einer Dosis von beispielsweise 0,001 - 50 mg pro kg Körpergewicht ein oder mehrmals am Tag als Bolus oder als Dauerinfusion verabreicht.

Ein solches PP4-enthaltendes Mittel wird vorzugsweise entweder in Form eines Lyophilisates gegebenenfalls mit Stabilisatoren und Zusätzen wie beispielsweise Albumin, Gelatine, Salze, Zucker und/oder Aminosäuren hergestellt oder aber in Form einer blutisotonischen, gegebenenfalls ein Bakterizid und Stabilisatoren, wie beispielsweise Albumin, Gelatine, Salze, Zucker und/oder Aminosäuren, enthaltenden wässrigen Lösung hergestellt. Die Lösung kann beispielsweise durch parenterale Injektion oder Perfusion verabreicht werden.

Auch ein gentechnisch hergestelltes PP4 kann im erfindungsgemäßen Sinn verwendet werden.

Zur Anwendung als Arzneimittel wird PP4 in größeren Mengen gebraucht.

PP4 kann aus einem Gewebeextrakt oder einer Lösung an eine hydrophobe Trägermatrix gebunden und durch eine anschließende Stufen- oder Gradientenelution weitestgehend von Verunreinigungen befreit werden.

Vor oder nach diesem Verfahren können noch gegebenenfalls andere bekannte Reinigungsverfahren wie Ionenaustauschchromatographie, Gelfiltration oder Adsorption an Calciumphosphat oder Hydroxylapatit durchgeführt werden.

In einer bevorzugten Verfahrensweise zur Reinigung von PP4 wird die PP4 enthaltende Lösung bei einem pH-Wert von 5.5 - 9.5, einer Temperatur von 0°C bis 40°C und nach Zusatz von Salzen, beispielsweise nach Zusatz von 50 - 250 g Ammoniumsulfat/l Lösung, Magnesiumsulfat oder 0.5 - 3 mol/l NaCl, LiCl oder KCl, mit einem wasserunlöslichen hydrophoben Adsorbens, beispielsweise einem Trägermaterial, das aliphatische, aromatische oder araliphatische Gruppen trägt, in Kontakt gebracht. Als Trägermaterial können beispielsweise [R]Sepharose, Agarose oder [R]Fractogel verwendet werden. Als aliphatische Gruppen werden vorzugsweise Alkylgruppen mit 1 bis 10 C-Atomen, beispielsweise der Butyl- oder Octyl-Rest, und weiterhin beispielsweise gegebenenfalls substituierte Phenyl- oder Phenalalanylreste oder Benzyl- oder substituierte Benzylreste verwendet. Das Adsorbens wird anschließend von der Lösung abgetrennt, gegebenenfalls mit einer Lösung, die beispielsweise 50 - 250 g/l Ammoniumsulfat oder 0.5 - 3 mol/l NaCl, LiCl oder KCl enthält, gewaschen, und das PP4 mit einer Flüssigkeit geringerer Ionenstärke, gegebenenfalls mittels eines Gradienten, eluiert.

In einer besonders bevorzugten Ausführungsform wird die PP4 enthaltende Lösung bei einem pH-Wert von 6 - 8 nach Zusatz einer 144 - 243 g Ammoniumsulfat/l enthaltenden Lösung mit beispielsweise Phenyl- oder Phenylalanyl-[R]Sepharose oder Agarose, ganz besonders bevorzugt aber bei pH 6.5 - 7.8 nach Zusatz einer 170 - 200 g Ammoniumsulfat/l enthaltenden Lösung mit Phenyl-Sepharose in Kontakt gebracht. Anschließend wird das Adsorbens von der Lösung abgetrennt, beispielsweise mit einer Lösung, die 176 - 196 g/l Ammoniumsulfat/l Lösung enthält, gewaschen und das PP4 mittels einer Lösung, die 114 - 144 g Ammoniumsulfat/l Lösung enthält, eluiert.

Weiterhin ist es wegen der Gefahr einer Übertragung von Krankheiten (Hepatitis, AIDS) zweckmäßig für eine Anwendung am Menschen bestimmte biologische Therapeutika zur Abtötung infektiöser Keime zu erhitzen. Dies bedeutet jedoch meist Aktivitätsverlust der biologischen Wirkstoffe.

Es wurde überraschenderweise gefunden, daß eine wässrige Lösung von PP4 in Gegenwart von Calciumionen sowie mindestens eines Mono- oder Oligosaccharides oder Zuckeralkohols und gegebenenfalls einer Aminosäure erhitzt werden kann, wobei die blutgerinnungshemmende Eigenschaft weitestgehend erhalten bleibt.

Gegenstand einer Teilanmeldung ist deshalb auch ein Verfahren zur Pasteurisierung einer PP4 enthaltenden Lösung, dadurch gekennzeichnet, daß diese Lösung in Gegenwart von Calciumionen sowie mindestens eines Mono- oder Oligosaccharids oder Zuckeralkohols und gegebenenfalls einer Aminosäure unter Bedingungen so erhitzt wird, daß pathogene Keime abgetötet werden.

In einer bevorzugten Verfahrensweise zur Pasteurisierung einer PP4 enthaltenden Lösung wird der pH-Wert der Lösung auf 5.0 - 9.5 eingestellt und 30 - 150 g/100 ml Lösung mindestens eines Mono- oder Oligosaccharides oder Zuckeralkohols und 0.005 - 2 mol/l Calciumionen sowie gegebenenfalls 0.1 - 1.5 mol/l einer Aminosäure zugesetzt. Die Calciumionen können beispielsweise in Form von Calciumsalzen, beispielsweise als $CaCl_2$ oder Calciumacetat zugeführt werden.

In einer ganz besonders bevorzugten Ausführungsform wird eine PP4 enthaltende Lösung, die einen pH-Wert von 6.5 - 8.5 besitzt, mit 100 - 150 g Saccharose/100 ml Lösung und 30 - 100 mmol/l $CaCl_2$ versetzt. Nach dem Lösen der Bestandteile kann die Lösung beispielsweise 1 bis 20 Stunden bei 40 - 80°C, vorzugsweise aber 8 - 15 Stunden bei 55 - 65°C, erhitzt werden. Der Gehalt an PP4 sollte nicht 20 μg/ml Lösung unterschreiten. Nach der Erhitzung kann die Lösung beispielsweise durch Konzentrierung, Dialyse, Sterilfiltration und/oder Lyophilisation weiterverarbeitet werden.

Ein nach dem oben beschriebenen Herstellungs- und Pasteurisierungsverfahren erhaltenes Präparat zeichnet sich besonders dadurch aus, daß PP4 weitestgehend von Verunreinigungen befreit und die blutgerinnungshemmende Wirkung weitestgehend vorhanden ist.

Das PP4 kann in üblicher Weise mit einem physiologisch unbedenklichen und pharmazeutisch geeigneten Träger in fester oder flüssiger Form und gegebenenfalls weiteren Zusatzstoffen wie Stabilisatoren und Hilfsstoffe zu einem Arzneimittel konfektioniert werden.

Gegenstand der Erfindung ist somit auch ein Verfahren zur Herstellung eines PP4-enthaltenden Arzneimittels zur Therapie und/oder Prophylaxe haemostatischer Störungen, dadurch gekennzeichnet, daß eine PP4-enthaltende Lösung hergestellt wird, der gegebenenfalls Zusätze und Stabilisatoren, wie beispielsweise Albumin, Gelatine, Salze, Zucker und/oder Aminosäuren zugesetzt werden, und diese Lösung gegebenenfalls in Form eines Lyophilisats zur Trockene gebracht wird.

Die Erfindung soll durch die nachstehenden Beispiele erläutert werden.

Beispiel 1

Gewinnung von PP4 aus Plazentagewebe und seine Pasteurisierung

2 kg tiefgefrorene Plazenta wurden in einem Gewebezerkleinerer homogenisiert. Das Homogenisat wurde 3 mal mit je 1 Liter physiologischer NaCl-Lösung gewaschen. Das Plazentagewebe wurde durch Zentrifugation von der überstehenden Lösung abgetrennt. Das gewaschene Plazentahomogenisat wurde in 3 Liter $^R$Triton X 100-haltigem Puffer A (20 mmol/l Tris HCl, 50 mmol/l NaCl, 2 g/100 ml $^R$Triton X 100 pH 7.4) suspendiert und über Nacht gerührt. Der Extrakt wurde durch Zentrifugation abgetrennt und das Gewebe nochmals mit 2 Liter Puffer A über Nacht extrahiert. Die beiden Extrakte wurden vereinigt (4.8 Liter) und mit Puffer B (20 mmol/l Tris/HCl, 50 mmol/l NaCl pH 7.5) auf 15 l verdünnt. Nach Zusatz von 0,75 g DEAE-$^R$Sephadex A 50/l Lösung wurde die Suspension 1 Stunde gerührt, das Adsorbat von der Lösung abgetrennt, mit 40 Liter Puffer B, danach mit 600 ml Puffer B, der 100 mmol/l NaCl enthielt, gewaschen und PP4 mit Puffer B, der 500 mmol/l NaCl enthielt, eluiert. Das Eluat (600 ml) wurde mit 176 g/l Ammoniumsulfat versetzt und entweder im Batch-Verfahren oder auf einer Säule mit 300 ml Phenyl-$^R$Sepharose behandelt.

Die Phenyl-$^R$Sepharose war in Puffer B, der 3/10 der Sättigungskonzentration Ammoniumsulfat enthielt, äquilibriert. Nach Waschen mit demselben Puffer wurde PP4 mit Puffer B, der 1/5 der Sättigungskonzentration Ammoniumsulfat enthielt, eluiert. Das PP4 wurde durch Erhöhen der Ammoniumsulfat-Konzentration auf 85 g/100 ml ausgefällt oder mittels Ultrafiltration konzentriert und über eine Gelfiltration an ACA 54 weitergereinigt. Die ACA 54 Säule (3 × 100 cm) war in Puffer B, der 500 mmol/l NaCl enthält, äquilibriert. Die PP4 enthaltenden Fraktionen wurden vereint.

Bei Bedarf kann sich noch eine Pasteurisierung wie in Beispiel 3 beschrieben, anschließen. Die Erhitzung kann aber auch auf einer anderen Stufe, wie des DEAE-$^R$Sephadex A50-Eluates oder des Eluates der Phenyl-$^R$Sepharose-Behandlung durchgeführt werden.

Beispiel 2

Bestimmung der blutgerinnungshemmenden Aktivität von PP4

Zur Bestimmung der blutgerinnungshemmenden Aktivität von nach Beispiel 1 hergestellten PP4 wurde die modifizierte Prothrombinzeit- (PT) und die modifizierte partielle Thromboplastinzeit (PTT) -Bestimmung verwendet.

a) modifizierte PT-Bestimmung

Zu 50 μl Standard-Human-Plasma wurden 150 μl Puffer A (20 mmol/l Tris/HCl, 142 mmol NaCl ph 7.5), 25 μl Probe oder Puffer B (20 mmol/l Tris/HCl, 500 mmol/l NaCl pH 7.5) und 25 μl Thromboplastinlösung zugesetzt. Nach einer dreiminütigen Inkubation bei 37°C wurde die Gerinnung durch Zugabe von

250 μl einer CaCl$_2$ enthaltenden Lösung C (10 mmol/l Tris/HCl, 80 mmol/l NaCl, 20 mmol/l CaCl$_2$ pH 7.9) gestartet und die Gerinnungszeit in einem Schnitger-Gross-Koagulometer bestimmt. Die Gerinnungszeit der Kontrolle mit Puffer B betrug 60 sec.

Abhängigkeit der Gerinnungszeit in der modifizierten PT-Bestimmung von dem Gehalt an PP4 in der Lösung

| PP4-Konzentrat (μg/ml) | 0 | 5 | 10 | 15 | 20 |
|---|---|---|---|---|---|
| modifiz. PT-Zeit (sec) | 60 | 68 | 89 | 111 | 132.5 |

b) modifizierte PTT-Bestimmung

Zu 100 μl Standard-Human-Plasma wurden 25 μl Probe und 75 μl Puffer A, der 100 mmol/l NaCl enthielt, gegeben, 3 min bei 37°C inkubiert, 50 μl RPathromtin-Reagenzlösung zugesetzt, 6 min bei 37°C weiter inkubiert, danach 50 μl Kaolin-Suspension zugemischt und nach weiteren 2 min bei 37°C die Gerinnung durch Zusatz von 100 μl CaCl$_2$-Lösung C gestartet.

Die Probe wurde zuvor gegen Puffer A, der 500 mmol/l NaCl enthielt, dialysiert. Eine Abfüllung RPathromtin-Reagenzlösung wurde in 1 ml 9 g/l NaCl-Lösung gelöst. Die Gerinnungszeit wurde in einem Schnitger-Gross-Koagulometer bestimmt. Die Gerinnungszeit der Kontrolle mit Puffer A betrug 70 sec.

Abhängigkeit der Gerinnungszeit in der modifizierten PTT-Bestimmung von dem Gehalt an PP4 in der Lösung

| PP4-Konzentrat (μg/ml) | 0 | 13 | 17.7 | 26 |
|---|---|---|---|---|
| modifiz. PTT-Zeit (sec) | 70 | 84 | 98 | 129 |

Beispiel 3

Pasteurisierung einer PP4 enthaltenden Lösung

500 ml einer PP4 enthaltenden Lösung (100 μg PP4/ml) wurden gegen 20 mmol/Tris/HCl, 150 mmol NaCl, pH 7,5 dialysiert. Anschließend wurden 100 g Saccharose/100 ml Lösung zugewogen. Nach dem vollständigen Lösen der Saccharose wurden 50 mmol/l CaCl$_2$ zugesetzt. Nach dem Erhitzen (10 h/60 °C) kann die Lösung gegen 20 mmol/l Tri-Na-Citrat, 60 mmol/l NaCl, 10 g/l Glycin, pH 7.0 dialysiert, sterilfiltriert und lyophilisiert werden.

Pasteurisierung von PP4 (100 μg/ml) in Lösung - Einfluß von Calcium-Ionen auf die Stabilität von PP4

| Modifizierte PT-Zeit (sec) | | |
|---|---|---|
| vor Erhitzung | Erhitzung ohne Calcium | Erhitzung mit 50 mmol/l Calcium |
| 68 | 60 | 68 |

**Patentansprüche**

**Patentansprüche für folgende Vertragsstaaten : AT, BE, CH, LI, LU**

1. Mittel zur Therapie oder Prophylaxe hämostatischer Störungen enthaltend PP4 und einen pharmazeutisch geeigneten Träger und/oder einen Stabilisator.

2. Mittel nach Anspruch 1, dadurch gekennzeichnet, daß es in Form einer blutisotonischen, gegebenenfalls ein Bakterizid enthaltenden wässrigen Lösung vorliegt.

3. Mittel nach Anspruch 1, dadurch gekennzeichnet, daß es in Form eines Lyophilisats, gegebenenfalls Zusätze und Stabilisatoren enthaltend, vorliegt.

**4.** Mittel nach Anspruch 1 in flüssiger Form und enthaltend 0,001 bis 100 mg PP4 pro ml.

**5.** Mittel nach Anspruch 1 in lyophilisierter Form.

**Patentansprüche für folgende Vertragsstaaten : GR, ES**

**1.** Verfahren zur Herstellung eines Mittels zur Therapie oder Prophylaxe hämostatischer Störungen, dadurch gekennzeichnet, daß PP4 und ein pharmazeutisch geeigneter Träger und/oder ein Stabilisator in eine Anwendung geeignete Form gebracht werden.

**2.** Verfahren nach Anspruch 1, dadurch gekennzeichnet, daß der Träger eine blutisotonische wässrige Lösung und ein gegebenenfalls vorhandener Stabilisator ein Bakterizid ist.

**3.** Verfahren nach Anspruch 1, dadurch gekennzeichnet, daß eine Lösung von PP4, die gegebenenfalls Zusätze und Stabilisatoren enthält, lyophilisiert wird.

**4.** Verfahren nach Anspruch 2, dadurch gekennzeichnet, daß die Lösung 0,001 bis 100 mg PP4 pro ml enthält.

**5.** Verfahren nach Anspruch 3, dadurch gekennzeichnet, daß als Stabilisatoren und Zusätze Albumin, Gelatine, Salze, Zucker und/oder Aminosäuren vorhanden sind.

**6.** Verfahren nach Anspruch 1, dadurch gekennzeichnet, daß der Träger eine blutisotonische wässrige Lösung ist und als Stabilisatoren und Zusätze Albumin, Gelatine, Salze, Zucker und/oder Aminosäuren vorhanden sind.

**Claims**

**Claims for the following Contracting States : AT, BE, CH, LI, LU**

**1.** An agent for the therapy or prophylaxis of disturbances of hemostatis, containing PP4 and a pharmaceutically suitable vehicle and/or a stabilizer.

**2.** An agent as claimed in claim 1, which is in the form of an aqueous solution which is isotonic with blood and contains, where appropriate, a bactericide.

**3.** An agent as claimed in claim 1, which is in the form of a lyophilisate which, where appropriate, contains additives and stabilizers.

**4.** An agent as claimed in claim 1, in liquid form and containing 0.001 to 100 mg of PP4 per ml.

**5.** An agent as claimed in claim 1, in lyophilized form.

**Claims for the following Contracting States : GR, ES**

**1.** A process for the preparation of an agent for the therapy or prophylaxis of disturbances of hemostasis, which comprises converting PP4 and a pharmaceutically suitable vehicle and/or a stabilizer into a form suitable for use.

**2.** The process as claimed in claim 1, wherein the vehicle is an aqueous solution which is isotonic with blood and the stabilizer present, where appropriate, is a bactericide.

**3.** The process as claimed in claim 1, wherein a solution of PP4 which, where appropriate, contains additives and stabilizers is lyophilized.

**4.** The process as claimed in claim 2, wherein the solution contains 0.001 to 100 mg of PP4 per ml.

**5.** The process as claimed in claim 3, wherein the stabilizers and additives present are albumin, gelatin, salts, sugars and/or amino acids.

**6.** The process as claimed in claim 1, wherein the vehicle is an aqueous solution which is isotonic with blood, and the stabilizers and additives present are albumin, gelatin, salts, sugars and/or amino acids.

**Revendications**

**Revendications pour les Etats contractants suivants : AT, BE, CH, LI, LU**

**1.** Médicament pour le traitement ou la prophylaxie de troubles hémostatiques, contenant la protéine PP4 et un véhicule pharmaceutiquement approprié et/ou un stabilisant.

**2.** Médicament selon la revendication 1, caractérisé en ce qu'il se trouve sous la forme d'une solution aqueuse isotonique par rapport au sang, contenant éventuellement un bactéricide.

**3.** Médicament selon la revendication 1, caractérisé en ce qu'il se trouve sous la forme d'un lyophilisat contenant éventuellement des additifs et des stabilisants.

**4.** Médicament selon la revendication 1, sous forme liquide et contenant de 0,001 à 100 mg de PP4 par ml.

**5.** Médicament selon la revendication 1, sous forme lyophilisée.

**Revendications pour les Etats contractants suivants : GR, ES**

**1.** Procédé pour la fabrication d'un médicament pour le traitement ou la prophylaxie de troubles hémostatiques, caractérisé en ce que l'on met sous une forme appropriée à l'utilisation de la protéine PP4 et un véhicule pharmaceutiquement approprié et/ou un stabilisant.

**2.** Procédé selon la revendication 1, caractérisé en ce que le véhicule est une solution aqueuse isotonique par rapport au sang, et un stabilisant éventuellement présent est un bactéricide.

**3.** Procédé selon la revendication 1, caractérisé en ce qu'une solution de PP4, qui contient éventuellement des additifs et des stabilisants, est lyophilisée.

**4.** Procédé selon la revendication 2, caractérisé en ce que la solution contient de 0,001 à 100 mg de PP4/ml.

**5.** Procédé selon la revendication 3, caractérisé en ce que, en tant que stabilisants et additifs, sont présents de l'albumine, de la gélatine, des sels, des sucres et/ou des aminoacides.

**6.** Procédé selon la revendication 1, caractérisé en ce que le véhicule est une solution aqueuse isotonique par rapport au sang et, en tant que stabilisants et additifs, sont présents de l'albumine, de la gélatine, des sels, des sucres et/ou des aminoacides.